# EUROPEAN PATENT APPLICATION

(11) **EP 0 921 195 A1**
(43) Date of publication of application: **09.06.1999**
(21) Application number: 98905812.8
(22) Date of filing: 10.03.1998
(51) Int. Cl.: C12N 15/29, C12N 15/63, C12N 5/10, A01H 1/00, A01H 5/00

(54) **ANTISENSE BASE SEQUENCES**

(30) Priority: 10.03.1997 JP 5520897
(71) Applicant: Japan Tobacco Inc., Tokyo 105-8422 (JP)
(72) Inventor: MARUTA, Yoshiyuki, Japan Tobacco Inc., Shizuoka 438-0802 r (JP); SAITO, Hideaki, pan Tobacco Inc., Shizuoka 438-0802 (JP)
(74) Representative: Reinhard - Skuhra - Weise & Partner
(86) International application number: JP9800955
(87) International publication number: WO9840489

(57) **Abstract**

The present invention provides a novel method to enhance an ability to suppress in vivo protein synthesis in a method of suppressing synthesis by use of an antisense nucleotide sequence. More specifically, the present invention provides an antisense nucleotide sequence comprising two or more successive repeats of the structural gene of interest or a fragment thereof in the antisense direction, an expression vector comprising said antisense nucleotide sequence, a host transformed by said expression vector and a method for suppressing the expression of a protein by use of said antisense nucleotide sequence.

SEQUENCE LISTING

(2) INFORMATION FOR SEQ ID NO: 1:
(i) SEQUENCE CHARACTERISTICS:
(A) LENGTH: 1644 base pairs
(B) TYPE: nucleic acid
(C) STRANDEDNESS: double
(D) TOPOLOGY: linear

(ii) MOLECULAR TYPE: cDNA to mRNA

(vi) ORIGINAL SOURCE:
(A) ORGANISM:

(ix) FEATURE: Glutelin A cDNA

(xi) SEQUENCE DESCRIPTION: SEQ ID NO: 1:
AAGTACGACG AAAATTCATT AGTACTACAA CAAC ATG GCA TCC ATA AAT CGC CCC 55
Met Ala Ser Ile Asn Arg Pro
1               5

ATA GTT TTC TTC ACA GTT TGC TTG TTC CTC TTG TGC AAT GGC TCT CTA 103
Ile Val Phe Phe Thr Val Cys Leu Phe Leu Leu Cys Asn Gly Ser Leu
        10                  15                  20

GCC CAG CAG CTA TTA GGC CAG AGC ACT AGT CAA TGG CAG AGT TCT CGT 151
Ala Gln Gln Leu Leu Gly Gln Ser Thr Ser Gln Trp Gln Ser Ser Arg
    25                  30                  35

CGT GGA AGT CCA AGA GAA TGC AGG TTC GAT AGG TTG CAA GCA TTT GAG 199
Arg Gly Ser Pro Arg Glu Cys Arg Phe Asp Arg Leu Gln Ala Phe Glu
40                  45                  50                  55

CCA ATT CGG AGT GTG AGG TCT CAA GCT GGC ACA ACT GAG TTC TTC GAT 247
Pro Ile Arg Ser Val Arg Ser Gln Ala Gly Thr Thr Glu Phe Phe Asp
                60                  65                  70

GTC TCT AAT GAG CAA TTT CAA TGT ACC GGA GTA TCT GTT GTC CGT CGA 295
Val Ser Asn Glu Gln Phe Gln Cys Thr Gly Val Ser Val Val Arg Arg
            75                  80                  85

GTT ATT GAA CCT AGA GGC CTT CTA CTA CCC CAT TAC ACT AAT GGT GCA 343
Val Ile Glu Pro Arg Gly Leu Leu Leu Pro His Tyr Thr Asn Gly Ala
        90                  95                  100

TCT CTA GTA TAT ATC ATC CAA GGG AGA GGT ATA ACA GGG CCA ACT TTC 391
Ser Leu Val Tyr Ile Ile Gln Gly Arg Gly Ile Thr Gly Pro Thr Phe
    105                 110                 115

CCA GGC TGT CCT GAG TCC TAC CAA CAA CAG TTC CAA CAA TCA GGC CAA 439
Pro Gly Cys Pro Glu Ser Tyr Gln Gln Gln Phe Gln Gln Ser Gly Gln
120                 125                 130                 135

GCC CAA TTG ACC GAA AGT CAA AGC CAA AGT CAA AAG TTC AAG GAT GAA 487
Ala Gln Leu Thr Glu Ser Gln Ser Gln Ser Gln Lys Phe Lys Asp Glu
                140                 145                 150

CAT CAA AAG ATC CAC CGT TTC AGA CAA GGA GAT GTA ATT GCA TTG CCT 535
His Gln Lys Ile His Arg Phe Arg Gln Gly Asp Val Ile Ala Leu Pro
            155                 160                 165

GCT GGT GTA GCT CAT TGG TGC TAC AAT GAT GGT GAA GTG CCA GTT GTT 583
Ala Gly Val Ala His Trp Cys Tyr Asn Asp Gly Glu Val Pro Val Val
        170                 175                 180

GCC ATA TAT GTC ACT GAT CTC AAC AAC GGT GCT AAT CAA CTT GAC CCT 631
Ala Ile Tyr Val Thr Asp Leu Asn Asn Gly Ala Asn Gln Leu Asp Pro
    185                 190                 195

AGG CAA AGG GAT TTC TTG TTA GCT GGA AAT AAG AGA AAC CCT CAA GCA 679
Arg Gln Arg Asp Phe Leu Leu Ala Gly Asn Lys Arg Asn Pro Gln Ala
200                 205                 210                 215

TAC AGG CGT GAG GTT GAG GAG CGG TCA CAG AAC ATA TTT AGT GGC TTT 727
Tyr Arg Arg Glu Val Glu Glu Arg Ser Gln Asn Ile Phe Ser Gly Phe
                220                 225                 230

AGC ACT GAA CTA CTT AGC GAG GCT CTT GGC GTA AGC GGC CAA GTG GCA 775
Ser Thr Glu Leu Leu Ser Glu Ala Leu Gly Val Ser Gly Gln Val Ala
            235                 240                 245

AGG CAG CTC CAA TGT CAA AAT GAC CAA AGA GGA GAA ATT GTC CGT GTC 823
Arg Gln Leu Gln Cys Gln Asn Asp Gln Arg Gly Glu Ile Val Arg Val
        250                 255                 260

GAA CAC GGG CTC AGT TTG CTG CAG CCA TAT GCA TCA TTG CAG GAG CAG 871
Glu His Gly Leu Ser Leu Leu Gln Pro Tyr Ala Ser Leu Gln Glu Gln
    265                 270                 275

GAA CAA GGA CAA GTG CAA TCA AGA GAG CGT TAT CAA GAA GGA CAA TAT 919
Glu Gln Gly Gln Val Gln Ser Arg Glu Arg Tyr Gln Glu Gly Gln Tyr
280                 285                 290                 295

CAG CAA AGT CAA TAT GGA AGT GGC TGC TCT AAC GGT TTG GAT GAG ACC 967
Gln Gln Ser Gln Tyr Gly Ser Gly Cys Ser Asn Gly Leu Asp Glu Thr
                300                 305                 310

TTT TGC ACC CTG AGG GTA AGG CAA AAC ATC GAT AAT CCT AAC CGT GCT 1015
Phe Cys Thr Leu Arg Val Arg Gln Asn Ile Asp Asn Pro Asn Arg Ala
            315                 320                 325

GAT ACA TAC AAT CCA AGA GCT GGA AGG GTT ACA AAT CTC AAC ACC CAG 1063
Asp Thr Tyr Asn Pro Arg Ala Gly Arg Val Thr Asn Leu Asn Thr Gln
        330                 335                 340

AAT TTC CCC ATT CTC AGT CTT GTA CAG ATG AGT GCA GTC AAA GTA AAT 1111
Asn Phe Pro Ile Leu Ser Leu Val Gln Met Ser Ala Val Lys Val Asn
    345                 350                 355

CTA TAC CAG AAT GCA CTC CTT TCA CCA TTT TGG AAC ATC AAC GCT CAC 1159
Leu Tyr Gln Asn Ala Leu Leu Ser Pro Phe Trp Asn Ile Asn Ala His
360                 365                 370                 375

AGC GTC GTG TAT ATT ACT CAA GGC CGT GCC CGG GTT CAA GTT GTC AAC 1207

Ser Val Val Tyr Ile Thr Gln Gly Arg Ala Arg Val Gln Val Val Asn
                380                 385                 390

AAC AAT GGA AAG ACA GTG TTC AAC GGC GAG CTT CGC CGC GGA CAG CTG 1255
Asn Asn Gly Lys Thr Val Phe Asn Gly Glu Leu Arg Arg Gly Gln Leu
            395                 400                 405

CTT ATT ATA CCA CAA CAC TAC GCA GTT GTA AAG AAG GCA CAA AGA GAA 1303
Leu Ile Ile Pro Gln His Tyr Ala Val Val Lys Lys Ala Gln Arg Glu
        410                 415                 420

GGA TGT GCT TAC ATT GCA TTC AAG ACC AAT CCT AAC TCT ATG GTA AGC 1351
Gly Cys Ala Tyr Ile Ala Phe Lys Thr Asn Pro Asn Ser Met Val Ser
    425                 430                 435

CAC ATT GCA GGA AAG AGT TCC ATC TTC CGT GCT CTC CCA AAT GAT GTT 1399
His Ile Ala Gly Lys Ser Ser Ile Phe Arg Ala Leu Pro Asn Asp Val
440                 445                 450                 455

CTA GCA AAT GCA TAT CGC ATC TCA AGA GAA GAG GCT CAG AGG CTC AAG 1447
Leu Ala Asn Ala Tyr Arg Ile Ser Arg Glu Glu Ala Gln Arg Leu Lys
                460                 465                 470

CAT AAT AGA GGA GAT GAG TTC GGT GCA TTC ACT CCA ATC CAA TAC AAG 1495
His Asn Arg Gly Asp Glu Phe Gly Ala Phe Thr Pro Ile Gln Tyr Lys
            475                 480                 485

AGC TAC CAA GAC GTT TAT AAT GCG GCA GAA TCC TCT TAG GTCGGCTTGC GG 1546
Ser Tyr Gln Asp Val Tyr Asn Ala Ala Glu Ser Ser Stop
        490                 495                 500

ATAAAGAATA ACTAAATAAA TAAATTGCAA GCAATTGTTT TGCTGCTATG TACTGTCCAG 1606

TCTTTCGACT AATGATGATA AAGCCTCTCT TTATCCTT 1644

(2) INFORMATION FOR SEQ ID NO: 2:
(i) SEQUENCE CHARACTERISTICS:
(A) LENGTH: 1634 base pairs
(B) TYPE: nucleic acid
(C) STRANDEDNESS: double
(D) TOPOLOGY: linear

(ii) MOLECULAR TYPE: cDNA to mRNA

(vi) ORIGINAL SOURCE:
(A) ORGANISM:

(ix) FEATURE: Glutelin B cDNA

(xi) SEQUENCE DESCRIPTION: SEQ ID NO: 2:
GTACAAATAG CT ATG GCG AGC TCC GTT TTC TCT CGG TTT TCT ATA TAC TTT 51
Met Ala Ser Ser Val Phe Ser Arg Phe Ser Ile Tyr Phe
1               5                   10

TGT GTT CTT CTA TTA TGC CAT GGT TCT ATG GCC CAG CTA TTT AAT CCC 99
Cys Val Leu Leu Leu Cys His Gly Ser Met Ala Gln Leu Phe Asn Pro
    15                  20                  25

AGC ACA AAC CCA TGG CAT AGT CCT CGG CAA GGA AGT TTT AGG GAG TGT 147
Ser Thr Asn Pro Trp His Ser Pro Arg Gln Gly Ser Phe Arg Glu Cys
30                  35                  40                  45

AGA TTT GAT AGA CTA CAA GCA TTT GAA CCA CTT CGG AAA GTG AGG TCA 195
Arg Phe Asp Arg Leu Gln Ala Phe Glu Pro Leu Arg Lys Val Arg Ser
                50                  55                  60

GAA GCT GGG GTG ACT GAG TAC TTC GAT GAG AAG AAT GAA TTA TTC CAG 243
Glu Ala Gly Val Thr Glu Tyr Phe Asp Glu Lys Asn Glu Leu Phe Gln
            65                  70                  75

TGC ACG GGT ACT TTT GTG ATC CGA CGT GTC ATT CAG CCT CAA GGC CTT 291
Cys Thr Gly Thr Phe Val Ile Arg Arg Val Ile Gln Pro Gln Gly Leu
        80                  85                  90

TTG GTA CCT CGA TAC ACA AAT ATT CCT GGC GTG GTC TAC ATC ATC CAA 339
Leu Val Pro Arg Tyr Thr Asn Ile Pro Gly Val Val Tyr Ile Ile Gln
    95                  100                 105

GGG AGA GGT TCT ATG GGT TTA ACC TTC CCC GGT TGC CCT GCG ACT TAC 387
Gly Arg Gly Ser Met Gly Leu Thr Phe Pro Gly Cys Pro Ala Thr Tyr
110                 115                 120                 125
CAG CAA CAA TTC CAA CAA TTT TCA TCT CAA GGC CAA AGT CAG AGC CAA 435
Gln Gln Gln Phe Gln Gln Phe Ser Ser Gln Gly Gln Ser Gln Ser Gln
                130                 135                 140

AAG TTT AGA GAT GAG CAC CAA AAG ATT CAT CAA TTT AGG CAA GGA GAC 483
Lys Phe Arg Asp Glu His Gln Lys Ile His Gln Phe Arg Gln Gly Asp
            145                 150                 155

ATT GTT GCT CTC CCA GCT GGT GTT GCA CAT TGG TTC TAC AAT GAT GGT 531
Ile Val Ala Leu Pro Ala Gly Val Ala His Trp Phe Tyr Asn Asp Gly
        160                 165                 170

GAT CGC CAT ATT GTT GCC GTA TAT GTT TAT GAC GTA AAC AAC AAC GCC 579
Asp Arg His Ile Val Ala Val Tyr Val Tyr Asp Val Asn Asn Asn Ala
    175                 180                 185

AAT CAG CTT GAA CCT AGG CAA AAG GAG TTC CTA TTA GCC GGC AAC AAC 627
Asn Gln Leu Glu Pro Arg Gln Lys Glu Phe Leu Leu Ala Gly Asn Asn
190                 195                 200                 205

AAT CGG GCT CAA CAA CAA CAA GTA TAT GGT AGC TCA ATT GAG CAA CAC 675
Asn Arg Ala Gln Gln Gln Gln Val Tyr Gly Ser Ser Ile Glu Gln His
                210                 215                 220

TCT GGG CAA AAC ATA TTC AGC GGA TTT GGT GTT GAG ATG CTA AGT GAG 723
Ser Gly Gln Asn Ile Phe Ser Gly Phe Gly Val Glu Met Leu Ser Glu
            225                 230                 235

GCT TTA GGC ATC AAC GCA GTA GCA GCA AAG AGG CTA CAG AGC CCA AAT 771
Ala Leu Gly Ile Asn Ala Val Ala Ala Lys Arg Leu Gln Ser Pro Asn
        240                 245                 250

GAT CAA AGA GGA GAG ATC ATA CAT GTG AAG AAT GGC CTT CAA TTG TTG 819

Asp Gln Arg Gly Glu Ile Ile His Val Lys Asn Gly Leu Gln Leu Leu
    255                 260                 265

AAA CCG ACT TTG ACA CAA CAG CAA GAA CAA GCA CAA GCA CAA GAT CAA 867
Lys Pro Thr Leu Thr Gln Gln Gln Glu Gln Ala Gln Ala Gln Asp Gln
270                 275                 280                 285

TAT CAA CAA GTT CAA TAC AGT GAA CGA CAG CAA ACA TCT TCT CGA TGG 915
Tyr Gln Gln Val Gln Tyr Ser Glu Arg Gln Gln Thr Ser Ser Arg Trp
                290                 295                 300

AAC GGA TTG GAG GAG AAC TTT TGC ACG ATC AAG GTG AGA GTA AAC ATT 963
Asn Gly Leu Glu Glu Asn Phe Cys Thr Ile Lys Val Arg Val Asn Ile
            305                 310                 315

GAA AAT CCT AGT CGT GCT GAT TCA TAC AAC CCA CGT GCC GGA AGG ATA 1011
Glu Asn Pro Ser Arg Ala Asp Ser Tyr Asn Pro Arg Ala Gly Arg Ile
        320                 325                 330

ACA AGT GTC AAT AGT CAG AAG TTC CCC ATC CTT AAC CTC ATC CAA ATG 1059
Thr Ser Val Asn Ser Gln Lys Phe Pro Ile Leu Asn Leu Ile Gln Met
    335                 340                 345

AGC GCT ACC AGA GTA AAC CTA TAC CAG AAT GCT ATT CTC TCG CCG TTC 1107
Ser Ala Thr Arg Val Asn Leu Tyr Gln Asn Ala Ile Leu Ser Pro Phe
350                 355                 360                 365

TGG AAC GTC AAT GCT CAT AGT TTG GTC TAT ATG ATT CAA GGG CGA TCT 1155
Trp Asn Val Asn Ala His Ser Leu Val Tyr Met Ile Gln Gly Arg Ser
                370                 375                 380

CGA GTT CAA GTC GTT AGT AAC TTT GGA AAG ACT GTG TTT GAT GGT GTC 1203
Arg Val Gln Val Val Ser Asn Phe Gly Lys Thr Val Phe Asp Gly Val
            385                 390                 395

CTT CGC CCA GGA CAA TTA TTG ATC ATT CCG CAA CAT TAT GCT GTC TTG 1251
Leu Arg Pro Gly Gln Leu Leu Ile Ile Pro Gln His Tyr Ala Val Leu
        400                 405                 410

AAG AAA GCA GAG CGT GAA GGA TGC CAA TAT ATC GCA ATC AAG ACA AAC 1299
Lys Lys Ala Glu Arg Glu Gly Cys Gln Tyr Ile Ala Ile Lys Thr Asn
    415                 420                 425

GCT AAC ACC TTC GTC AGC CAC CTT GCA GGG AAA AAC TCA GTA TTC CGT 1347
Ala Asn Thr Phe Val Ser His Leu Ala Gly Lys Asn Ser Val Phe Arg
430                 435                 440                 445

GCC TTG CCA GTT GAT GTA GTC GCT AAT GCG TAT CGC ATC TCA AGG GAG 1395
Ala Leu Pro Val Asp Val Val Ala Asn Ala Tyr Arg Ile Ser Arg Glu
                450                 455                 460

CAA GCC CGA AGC CTC AAG AAC AAC AGG GGA GAA GAG CAC GGT GCC TTC 1443
Gln Ala Arg Ser Leu Lys Asn Asn Arg Gly Glu Glu His Gly Ala Phe
            465                 470                 475

ACT CCT AGA TTT CAA CAA CAA TAC TAC CCA GGA TTA TCG AAT GAG TCC 1491
Thr Pro Arg Phe Gln Gln Gln Tyr Tyr Pro Gly Leu Ser Asn Glu Ser

480                    485                    490

GAA AGC GAG ACC TCA GAG TAA TGTAATGTAA TTGAGAACTA GTATCGGCGT AGAG 1546
Glu Ser Glu Thr Ser Glu Stop
    495                 500

TAAAATAAAA CACCACAAGT ATGACACTTG GTGGTGATTC TGTTCGATAT CAGTACTAAA 1606

TAAAGGTTAC AAACTTCTTA ATTTTCCT                                    1634

(2) INFORMATION FOR SEQ ID NO: 3:
     (i) SEQUENCE CHARACTERISTICS:
          (A) LENGTH: 30 base pairs
          (B) TYPE: nucleic acid
          (C) STRANDEDNESS: single
          (D) TOPOLOGY: linear

    (ii) MOLECULAR TYPE: Other nucleic acids, synthetic DNA

    (xi) SEQUENCE DESCRIPTION: SEQ ID NO: 3:
AGTGGGCTGC AGGAATTCGA TATCAAGCTT                    30

(2) INFORMATION FOR SEQ ID NO: 4:
     (i) SEQUENCE CHARACTERISTICS:
          (A) LENGTH: 20 base pairs
          (B) TYPE: nucleic acid
          (C) STRANDEDNESS: single
          (D) TOPOLOGY: linear

    (ii) MOLECULAR TYPE: Other nucleic acids, synthetic DNA

    (xi) SEQUENCE DESCRIPTION: SEQ ID NO: 4:
AGTACATAGC AGCAAAACAT                               20

(2) INFORMATION FOR SEQ ID NO: 5:
     (i) SEQUENCE CHARACTERISTICS:
          (A) LENGTH: 25 base pairs
          (B) TYPE: nucleic acid
          (C) STRANDEDNESS: single
          (D) TOPOLOGY: linear

    (ii) MOLECULAR TYPE: Other nucleic acids, synthetic DNA

    (xi) SEQUENCE DESCRIPTION: SEQ ID NO: 5:
TACATAGCTT TAACTGATAA TCTGA                         25

(2) INFORMATION FOR SEQ ID NO: 6:
     (i) SEQUENCE CHARACTERISTICS:
          (A) LENGTH: 20 base pairs
          (B) TYPE: nucleic acid
          (C) STRANDEDNESS: single
          (D) TOPOLOGY: linear

    (ii) MOLECULAR TYPE: Other nucleic acids, synthetic DNA

    (xi) SEQUENCE DESCRIPTION: SEQ ID NO: 6:

AGTACATAGC AGCAAAACAT 20

## Description

### BACKGROUND OF THE INVENTION

This invention relates to a technique for suppressing protein synthesis using antisense nucleotide sequences, particularly to a technique capable of enhanced suppression of protein synthesis.

### PRIOR ART

It is known that functional RNAs such as messenger RNA which provides information on protein synthesis are suppressed by RNAs having complementary nucleotide sequences to the aforementioned RNA, which are collectively referred to as antisense RNAs. Research is under way to create plants wherein antisense RNA is introduced artificially by gene recombinant technology. Genes for expressing antisense RNAs are constructed in such a manner that a sequence corresponding to a partial or full length of a DNA sequence (cDNA or genomic DNA) coding for a protein of interest is connected downstream of a promoter in the antisense direction.

While various antisense techniques have been proposed, the following are worth mentioning:
(1) Recombinant petunia was prepared that produced an antisense RNA against RNA of a chalcone synthase gene participating in the synthesis of flower pigments, and the recombinant petunia presented a different flower color than the wild type (EP 34-1885A);
(2) The expression of a polygalacturonase gene, a key factor in tomato fruit losing its firmness, was suppressed by an introduced antisense RNA to create tomatoes that could be preserved for a longer period than the wild type (EP 891115A);
(3) Melton et al. used a full-length sequence of β-globin cDNA as an antisense gene (Proc. Natl. Acad. Sci. USA 82:144-148 (1985));
(4) Stockhaus et al. used as an antisense gene a sequence corresponding to the full length of a 10 kD protein cDNA which takes part in the photo-system of photosynthesis (the EMBO Journal 9:3013-3021 (1990));
(5) Alexander et al. used as an antisense gene a sequence corresponding to the full length of chalcone synthase cDNA which takes part in the synthesis of flower pigments (Nature 333:866-869(1988));
(6) Hamilton et al. used a sequence corresponding to the full length of ethylene synthase (ACC-oxidase) cDNA as an antisense gene (Nature 346:284-287 (1990)); and
(7) Smith et al. used a sequence corresponding to a partial length of polygalacturonase cDNA as an antisense gene (Nature 334:724-726 (1988)).

Thus, in the conventional antisense-related technology, a nucleotide sequence corresponding to a partial or full length of the nucleotide sequence coding for a protein of interest is simply inserted as an antisense gene in the reverse direction at a downstream of a promoter.

While several reports have been published which propose methods for reducing the content of a protein of interest using antisense RNA, there is no report of success in reducing protein synthesis when the protein is one of those occurring abundantly in vivo as exemplified by the storage proteins in plant seeds. In order to ensure that the content of a protein occurring abundantly in vivo is reduced using an antisense RNA, the latter must be provided in a large amount at the site of its syntheses. One possible means of satisfying this need is to introduce the antisense gene in multiple copies; however, if homozygote is desired as in the case where the host is a plant, the problem is that a great amount of work is required to fix a gene introduced in multiple copies by self-fertilization. Another possible means is to enhance the activity of a promoter that expresses an antisense gene of interest, but this also is not easy to accomplish.

The antisense genes reported thus far do not provide an easy way to reduce the content of proteins that occur abundantly in vivo.

### SUMMARY OF THE INVENTION

It is generally believed that the degree of suppression of protein synthesis increases with the increasing expression of associated antisense genes (Melton D.A. et al. (1985) Proc. Natl. Acad. Sci. USA 82, 144-148; and Ecker J.R. et al. (1986) Proc. Natl. Acad. Sci. USA 83, 5372-5376). As already mentioned, the expression of an antisense gene could be increased by, for example, enhancing the activity of a promoter used in expressing the antisense gene or by introducing it in an increased copy number. However, both methods are subject to the problems described above. The purpose of the present invention is to provide an antisense gene that can not only suppress the synthesis of a protein in vivo more efficiently but also lower even the content of a protein that occurs abundantly in vivo.

If a nucleotide sequence corresponding to a full length of a structural gene of interest is introduced in the antisense direction, the effectiveness in reducing the synthesis of a protein of interest is indeed increased to some extent. However, according to this method in which a nucleotide sequence corresponding to the full length of the structural gene of interest is introduced in the antisense direction, it is possible that an unknown open reading frame will appear, thereby inducing the expression of an unexpected protein in the host. Particularly in the case of edible plants, safety to humans and changes in taste are two important factors that must be considered seriously. The problem of the expression of an unexpected protein can be solved by suppressing it using a sequence corresponding to a portion of the structural gene, namely, a partial sequence which lacks an open reading frame or one from which an open reading frame has been deleted artificially. However, if a part of the structural gene of interest is introduced as a single unit, the effectiveness in reducing the synthesis of the protein by the structural gene will not be attained at the intended level. Under the circumstances, the present inventors conducted intensive studies in order to develop a method for solving the aforementioned problems by reducing the synthesis of a protein of interest without expressing any unknown protein. As a result, the inventors found a method wherein an antisense nucleotide sequence is prepared so that it comprises a plurality of the structural gene of interest or a fragment thereof linked in succession, and said sequence is introduced into a genomic gene in a target cell; the present invention has been accomplished on the basis of this finding.

### BRIEF DESCRIPTION OF THE DRAWINGS

Fig. 1 shows schematically the structures of genes used in the in vitro RNA syntehsis;
Fig. 2 shows electrophoretic patterns in the analysis of translation products in a wheat germ extract;
Fig. 3 is a graph showing the changes in the amount of glutelin synthesis;
Fig. 4 shows schematically the structure of a plasmid vector used in transformation;
Fig. 5 shows an electrophoretic pattern in the PCR analysis of transformants having a sequence comprising 8 repeats of glutelin A antisense gene introduced therein;
Fig. 6 shows an electrophoretic pattern in the PCR analysis of transformants having a full length of glutelin A antisense gene introduced therein;
Fig. 7 shows an electrophoretic pattern in the northern analysis of transformants having a sequence comprising 8 repeats of glutelin A antisense gene introduced therein; and
Fig. 8 shows schematically the structure of the plasmid vector used in the transformation.

### DETAILED DESCRIPTION OF THE INVENTION

The present inventors have found that an antisense nucleotide sequence may be formed such that it comprises two or more units of a structural gene of interest or a sequence representing a part of said structural gene in succession in the antisense direction, and said sequence is introduced into a genomic gene in a target cell so as to suppress the intracellular expression of a protein encoded by the structural gene effectively. The present invention was accomplished on the basis of this finding.

Thus, according to one aspect of the invention, there is provided an antisense nucleotide sequence in which two or more units of a structural gene of interest or a sequence representing a part of said structural gene are linked in succession in the antisense direction. An antisense RNA transcribed from the antisense nucleotide sequence of the invention comprises in the molecule two or more successive repeats of an RNA sequence complementary to the part of mRNA coding for a protein of interest. Therefore, the transcribed antisense RNA will form a pair with the strand of the mRNA coding for the protein of interest, thereby enhancing the possibility of suppressing the expression of said protein in vivo. Accordingly, the antisense RNA comprising the repeats of a complementary RNA sequence is more effective in suppressing the synthesis of the protein of interest than antisense RNA having no such repeats of a sequence unit.

According to further aspects of the invention, there are provided an expression vector having the antisense nucleotide sequence of the invention, a transformant obtained by transformation with said expression vector, and a method of suppressing the intracellular expression of a protein encoded by a structural gene which includes the step of introducing the antisense nucleotide sequence of the invention into a genomic gene in a target cell.

The invention will now be described in detail.

According to a first aspect of the invention, there is provided an antisense nucleotide sequence in which two or more repeats of a structural gene of interest or a sequence representing a part of said structural gene are linked in succession in the antisense direction.

The term "a structural gene of interest" as used herein means a gene coding for a protein whose expression is desired to be suppressed. The protein to be under expressed is not limited to any particular type as long as it is produced in vivo; however, proteins that are expressed abundantly in vivo are preferred targets since the antisense nucleotide sequence of the invention will work more effectively. Examples of such proteins are storage proteins in plant seeds. More specific examples include glutelin, prolamin, globulin, albumin and so forth in cereals; particularly, rice glutelin, wheat glutenin, maize zein and barley hordein occur abundantly in seeds. Other examples include soybean conglycinin, kindney bean phaseolin, as well as potato patatin and sweet potato sporamin.

In the Examples that are given hereinafter, glutelins A and B, storage proteins in rice, were used but they are just intended as illustrative embodiments of the invention.

In the invention, the entire sequence of a particular structural gene may be employed or, alternatively, a sequence representing a part of said gene may be employed. In the latter case, a sequence from around the 5' side is preferably used because a complementary sequence corresponding to the initiation site of the structural gene is more effective in suppressing the expression of the protein of interest.

If a partial sequence is used, its size is not limited to any particular length; generally, a sequence length of at least 45 nucleotides is preferred, with a length of at least 300 nucleotides being particularly preferred (Tada et al. (1996) Breeding Science 46, 403-407).

The antisense nucleotide sequence of the invention is such that multiple units of the above-described sequence are linked in succession in the antisense direction.

The term "antisense direction" as used herein means such a direction that at least a part of the antisense DNA sequence is oriented to provide a region which is complementary to the genomic DNA of host cells. The transcript from the complementary gene has a sequence that is complementary to the RNA sequence, particularly mRNA, which is endogenous in the host.

The term "multiple units" as used herein means at least two units, preferably at least 4 units, more preferably at least 8 units.

The term "in succession" as used herein means that adjacent units of the sequence are not interrupted by any other nucleotide or that any intervening sequence is not a promoter or other sequences that will cause significant effects on the expression of the structural gene.

In the invention, multiple units of the antisense nucleotide sequence can be linked by any customary methods used in genetic engineering technology and appropriate methods may be applied without any particular limitations.

In an exemplary method, a structural gene of interest is cloned in a plasmid and the sequence containing a full length of the structural gene or a fragment thereof, containing a part of the structural gene (such sequence or a fragment thereof is referred to as an insert) is excised from a plasmid. Different restriction sites are introduced at the two terminal ends of the excised insert by a suitable means such as the addition of linkers. In this case, it is important that the sequence at the cloning site of the vector (e.g. plasmid vector) in the subsequent cloning step is taken into consideration, in order to ensure that the restriction sites at the two terminal ends of the insert may be designed in such a way that the resulting plasmid contains the structural gene of interest in the antisense direction. Subsequently, the vector having the different restriction sites at the two ends and the insert having the corresponding restriction sites at the two terminal ends are ligated to produce a gene construct containing one antisense nucleotide sequence.

In the next step, a construct is linearized by treatment with a restriction enzyme and different restriction sites are introduced at the two terminal ends. In the same manner as described in the preceding paragraph, another insert containing a full or partial length of the structural gene is excised and the corresponding restriction sites are introduced at the two terminal ends of the insert. The resulting sequence is ligated with the linearizied construct to thereby produce a gene construct containing two antisense sequences.

The same procedure may be repeated as many times as is required to produce a gene construct containing a desired number of antisense nucleotide sequences. It is also possible to start from a gene construct containing two antisense nucleotide sequences, and excise a fragment containing the antisense sequences to be used as an insert thereby to obtain a gene construct containing four antisense sequences through one ligation step. Similarly, starting from a gene construct containing four antisense sequences, a gene construct containing eight antisense sequences can be prepared through one ligation step.

In one embodiment, the invention also provides an antisense nucleotide sequence which comprises two or more different types of structural genes or sequences representing a part of them, as the structural genes of interest. Stated specifically, if different types of structural genes are to be underexpressed simultaneously, a transforming gene construct may be so designed that it comprises a linkage of multiple units of transforming gene constructs each comprising an antisense nucleotide sequence of the invention that is placed downstream of a promoter and followed by a terminator. Since each of said gene construct units comprises an antisense sequence of a different type of structural gene or a part thereof oriented in the antisense direction, multiple structural genes can be suppressed from expression by use of a single transforming gene construct.

According to a second aspect of the invention, there is provided an expression vector having the antisense nucleotide sequence of the inveniton.

In constructing the expression vector, regulator such as a promoter and a terminator, and a transformation vector may be appropriately selected from among those which are customarily used in genetic engineering and they are not limited to any particular types.

Exemplary promoters include glutelin promoter, conglycinin promoter, phaseolin promoter, ADH promoter, heat shock protein promoter, a tissue specific promoter, a promoter associated with fruit ripening, prolamin promoter, RUBP carboxylase small subunit promoter cauliflower mosaic virus promoter, and so forth.

Exemplary terminators include nopalin synthase terminator, cauliflower mosaic virus terminator, and so forth.

Exemplary vectors include pUC plasmid vector, pBR plasmid vector, Ti plasmid vector, Ri plasmid vector, and so forth.

According to a third aspect of the invention, there is provided a transformant produced by transformation with the expression vector of the invention.

The organisms to be transformed are not limited to any particular types and may be selected as appropriate from among plants, animals, microorganisms and so forth. In the present invention, transformants are preferably plants, in particular, higher plants such as monocotyledons and dicotyledons; particularly preferred are cereals (e.g. rice, wheat, maize and barley), soybean, kidney bean and potato and sweet potato.

According to a fourth aspect of the invention, there is provided a method in which intracellular expression of proteins encoded by structural genes is suppressed by introducing the antisense nucleotide sequence of the invention into a genomic gene in a target cell.

The method of introducing the antisense nucleotide sequence of the invention into a genomic gene in a target cell is not limited in any particular way and a suitable transforming technique may be employed depending upon a specific object.

For example, the antisense nucleotide sequence of the invention may be introduced into genomic genes in plant cells by the use of Agrobacterium tumefaciens, electroporation into protoplasts, liposome fusion, microinjection, and so forth.

Plant cells into which the antisense nucleotide sequence has been sucessfully introruced can be selected by a suitable technique such as screening of antibiotic resistant cells. The thus transformed plant cells may be cultured for regeneration into intact plants. The regenerated plant can be fixed in a variety by customary breeding techniques.

The antisense nucleotide sequence may be introduced into genomic genes in animal cells by electroporation, liposome fusion, microinjection and other suitable methods.

The antisense nucleotide sequence may be introduced into genomic genes in microorganism cells by the calcium method, electroporation and other suitable methods.

The following examples are provided for the purpose of further illustrating the present invention but are in no way to be taken as limiting.

### EXAMPLES

In the examples, an attempt was made to suppress the storage proteins of rice, glutelin, by way of antisense RNAs in order to reduce the glutelin content in rice endosperm. With regard to glutelin, rice contains 10 or more genes in the genome coding for either glutelin A or glutelin B. The homology of their nucleotide sequences is 65%. Reducing the glutelin content of rice provides the rice with better quality for brewery.

A brief summary of the steps employed in the examples is as follows. Where no specific indication is made, the methods are in accordance with Maniatis T. et al. (Molecular Cloning, Cold Spring Harbor (1982)).
(a) The full length cDNAs of the rice storage proteins, glutelin A and glutelin B were isolated (Okita et al. (1989) Journal. Biochemical Chemistry 264: 12573-12581). The entire nucleotide sequences of the cDNAs of glutelin A and glutelin B are shown as SEQ ID NO: 1 and SEQ ID NO: 2, respectively.
(b) A plurality of the 312 bps sequence from the 5' upstream of the full length cDNA of glutelin A were linked in the antisense direction.
(c) The antisense gene comprising the linkage was used as the template for in vitro transcription to prepare the corresponding antisense RNA.
(d) The antisense RNA was mixed with the sense RNA of glutelin A and the mixed solution was used in in vitro translation to study the amount of glutelin A synthesis.
(e) The first intron of castor bean catalase gene (Ohta S. et al. (1990) Plant Cell Physiol. 31, 805-813) was introduced downstream to the glutelin promoter; and 8 repetitions of the 312 bps sequence from the 5' upstream of the full length cDNA of glutelin A were placed downstream to said intron. A terminator of nopaline synthase was further added to provide an expression plasmid vector. Similarly, an antisense gene comprising 8 repetitions of the 287 bps sequence from the 5' upstream of the full length cDNA of glutelin B was prepared, which was then introduced into said expression plasmid vector.
(f) The expression plasmid vector was used to transform a rice plant and the glutelin content in the rice seeds was studied.

### Example 1. In vitro effects of tandem-repeated antisense RNA

### (1) RNA synthesis in vitro

### (1-1) Synthesis of sense mRNA for glutelin A

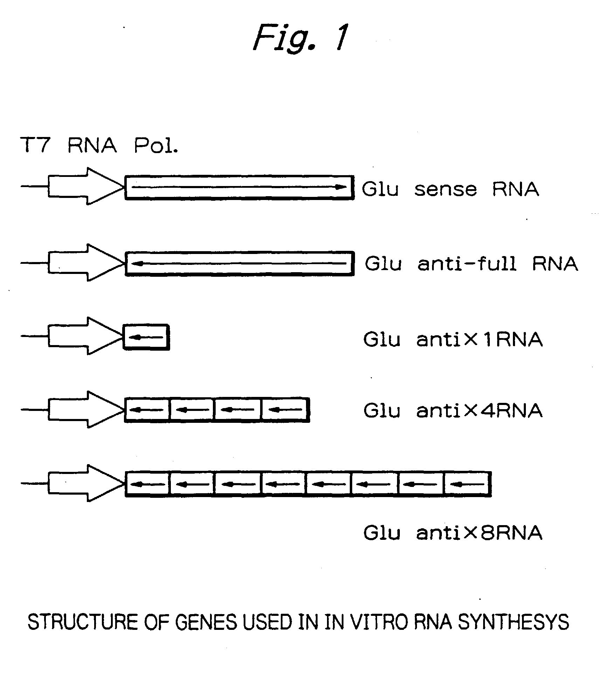

A full-length glutelin A cDNA was inserted, in the sense direction, into the multiple cloning site *Eco*RI/*Bam*H1 that is located downstream of the T7 promoter in Bluescript plasmid(product from Toyobo Co.) (Fig. 1, Glu sense RNA). Transcription was carried out in a reaction mixture containing the plasmid described above (5 µg of DNA in 50 µl) by adding thereto 20 mM each of ATP, CTP, and GTP (4 µl), 2.5 mM GTP (1 µl), 5 mM Cap analogue (5 µl), 0.5 M DTT (1 µl), RNase inhibitor (50 units), and T7 RNA polymerase (10 units). The reaction mixture was incubated for 30 min at 37°C and further 4.5 hr at 37°C after addition of 20 mM GTP (1 µl).

### (1-2) Synthesis of glutelin A antisense RNA

A full-length glutelin A cDNA was inserted, in the antisense direction, to the multiple cloning site *Bam*HI/*Eco*RI that is located downstream of the T7 promoter in Bluescript plasmid(product from Toyobo Co.) (anti-full RNA). Similarly, a 312 bps fragment from the 5'-end of glutelin A cDNA sequence was inserted in Bluescript plasmid in the antisense direction, singly (anti x 1 RNA), 4-times repeatedly (anti x 4 RNA), or 8-times repeatedly (anti x 8 RNA) (Fig. 1) as follows. First, a *Xba*I linker (CTCTAGAG) was added to the *Stu*I site that existed at 312 bps downstream from the 5'-end of glutelin A cDNA. Plasmid anti x 1 RNA was constructed by inserting the *Bam*HI/*Xba*I fragment thereof into the multiple cloning site *Xba*I/*Bam*HI, which is located downstream of the T7 promoter, in Bluescript plasmid (product of Toyobo Co.). Next, a *Bam*HI linker (CGGATCCG) was added to the *Stu*I site which is located 312 bps downstream from the 5'-end of glutelin A cDNA, and the *Bam*HI/*Sca*I fragment separated therefrom was inserted into the *Bam*HI/*Sma*I site of the above plasmid anti x 1 RNA, to provide plasmid anti x 2 RNA. The plasmid was digested at the *Xba*I site, treated with DNA polymerase to fill in the sticky ends, and subsequently treated with *Eco*RI to provide a fragment containing two of said 5'end sequence of appoximately 312 bps. Separately, plasmid anti x 2 RNA was digested at the PstI site, filled in by DNA polymerase, and cleaved with EcoRI. Plasmid anti x 4 RNA was constructed by inserting the above fragment into this *Eco*RI site. Similarly, plasmid anti x 8 RNA was constructed by inserting the *Xho*I fragment which was removed from plasmid anti x 4 RNA after the *Xba*I cut site was filled in by treatment with DNA polymerase into the *Hin*cII/*Xho*I site of plasmid anti x 4 RNA.

Transcription was carried out in a reaction mixture that contained the plasmid (5 µg of DNA), a mixture of 20 mM each of ATP, CTP, UTP, and GTP (5 µl), 0.5 M DTT (1 µl), RNase inhibitor (50 units), and T7 RNA polymerase (10 units), by incubation at 37°C for 5 hr,.

### (2) In vitro translation reaction by using wheat germ extract

Translation experiments were conducted by using wheat germ extract (Amersham). To the Glu sense RNA (2 picomoles) that had translation activity to glutelin was added antisense RNA against glutelin sense RNA as follows: anti-full RNA, anti x 1 RNA, anti x 4 RNA, or anti x 8 RNA, to provide an amount of 0.2, 0.5, 1, and 2 picomoles, respectively. Translation reaction was carried out at 30°C for 1 hr after addition of 15 µl of wheat germ extract to a mixture containing 2 µl of 1 mM amino acid mixture (19 amino acids lacking methionine) and 0.5 µl of ³⁵S-methionine (1,000 Ci/mmol). The reaction was terminated by addition of 20% SDS (2.5 µl) followed by heating at 95°C for 5 min. The translation products were separated by electrophoresis on 13% polyacrylamide gel. The radioactivity was detected and measured by using Imaging analyzer (Fuji Film Co.).

### (3) In vitro analysis of the effect of antisense RNA

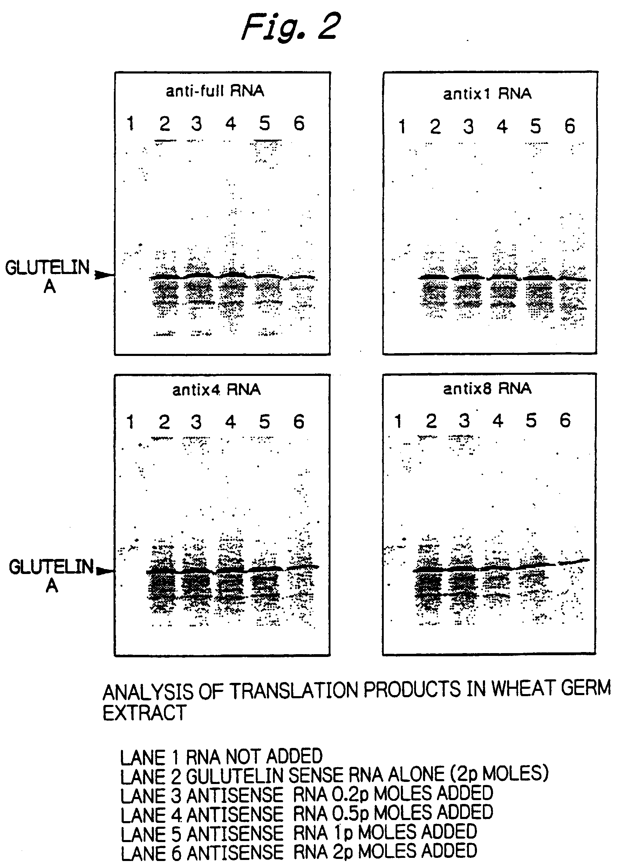

Amount of the translation product synthesized by wheat germ extract, in the presence of the antisense RNAs, was analyzed by using Imaging analyser (Fig. 2). No band appeared if no RNA was added (lane 1), while the glutelin band appeared when the glutelin sense RNA alone was added (lane 2). However, the intensity of the glutelin band was smaller as the moles of the antisense RNA added was greater (lanes 3-6) .

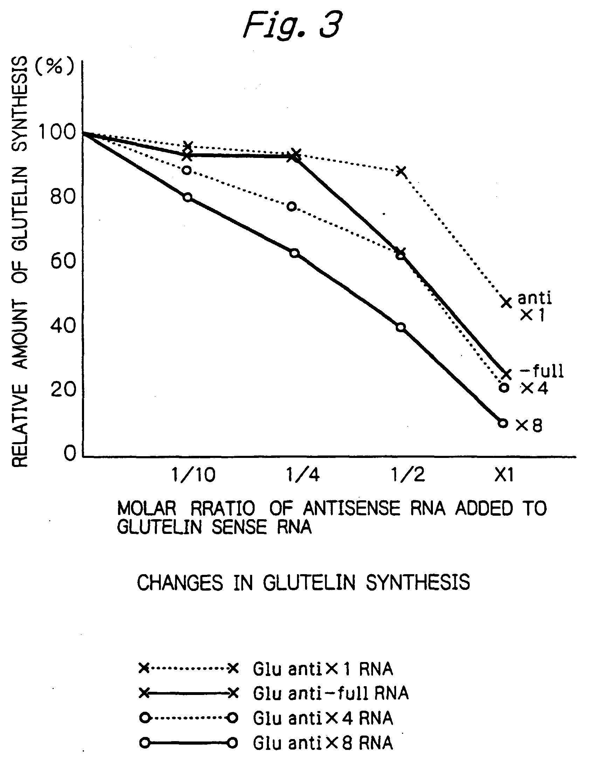

From the radioactivity of the glutelin bands, amounts of glutelin synthesized under the given conditions were determined. The graph (Fig. 3) shows the results for the amounts of glutelin synthesized under the given conditions, in percentage as compared with the control which contained no antisense RNA. The decrease of glutelin depended on the number of the linked 5'-upstream 312 bps sequences of the glutelin A cDNA. Such decrease was larger in cases in which the antisense RNA comprised 4-tandem repeats or 8-tandem repeats of the 5'-upstream 312 bps sequence than in the case in which the full-length glutelin antisense RNA was used.

### Example 2. Production and analysis of transgenic plants transformed by glutelin A antisense gene

### (1) Construction of 8-tandem repeats of glutelin A antisense gene for transformation

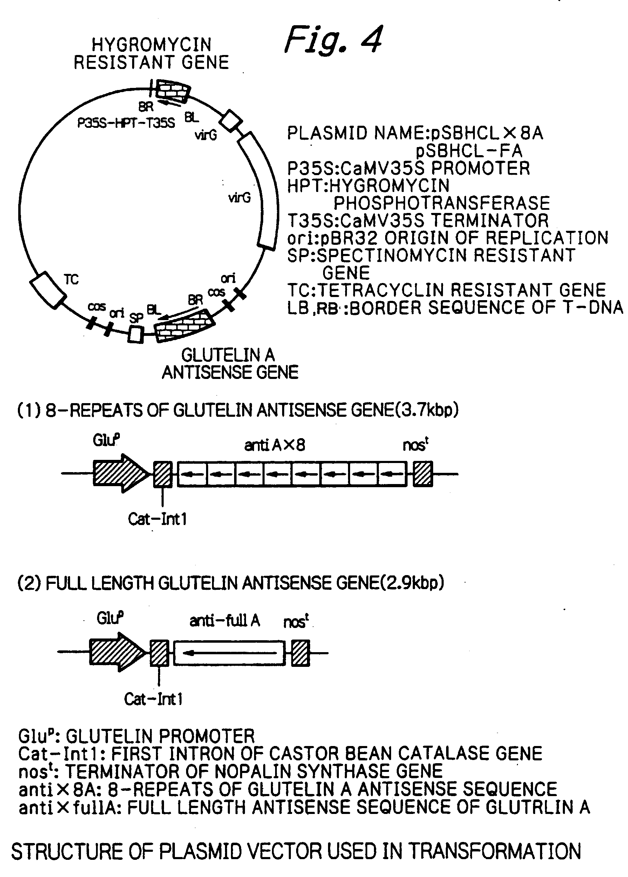

A *Sma*I/*Xba*I fragment containing the first intron of the castor bean catalase gene (Ohta S. et al. (1990) Plant Cell Physiol. 31, 805-813) was inserted into the *Sca*I/*Xba*I site downstream of the glutelin promoter (Takaiwa et al. (1987) FEBS Lett. 221:43-47), and then a *Xba*I/*Sac*I fragment containing 8-tandem repeats of the 5'-upstream 312 bps from the glutelin A cDNA was inserted into its downstream, in the antisense direction. A *Sca*I/*Eco*RI fragment containing the terminator of nopalin synthase (Depicker et al. (1982) J. Mol. Appl. Genet. 561-573) was joined to it and then the so formed antisense gene was inserted into a plasmid vector having a hygromycin resistance gene, to generate pSBHCI x 8A (Fig. 4). The intron inserted in the plasmid enhances the expression of glutelin A antisense gene.

### (2) Construction of a full-length glutelin A antisense gene for transformation

A *Sma*I/*Xba*I fragment containing the first intron of the castor bean catalase gene (Ohta S. et al. (1990) Plant Cell Physiol. 31, 805-813) was inserted into the *Sca*I*/Xba*I site downstream of the glutelin promoter, and then the *Sca*I/*Xba*I fragment containing glutelin A cDNA was inserted into its downstream, in the antisense direction. A *Sca*I/*Eco*RI fragment containing the terminator of nopalin synthase was joined to it and then the so formed antisense gene was inserted into a plasmid vector having a hygromycin resistance gene, to generate pSBHCI-FA (Fig. 4).

### (3) Transformation to rice plants

A variety of the rice plant, "Tsukinohikari", was transformed by *Agrobacterium tumefaciens* LBA4404 which harbored the above plasmid pSBHCI x 8A or pSBHCI-FA, according to the method of Hiel et al. (Plant J. 6, 271-282 (1994)). Transformed calluses were selected in the presence of hygromycin as described by Hiel et al. (*ibid*).

### (4) Preparation of DNA and RNA

DNA of transgenic plants was prepared from redifferentiated seedlings before they were transferred to pot cultivation in a closed greenhouse.

RNA was prepared by the SDS-phenol method (Uchimiya et al. Manual for plant genetic engineering, Kodansha Co., Tokyo, Japan).

### (5) PCR and Northern analyses of transformed plants

Transformation was confirmed by detecting the glutelin antisense gene after the gene was amplified by PCR. Genomic DNA (120 ng), four dNTPs (200 µM each), primers (10 pmol/reaction), and *Taq* DNA polymerase (Takara Biomedicals Co.) (1 unit) were mixed. The mixture was heated for 2 min at 94°C, 50°C, and 72°C, respectively, in the first cycle, and subsequently 1 min at each temperature for 34 cycles. For amplification of the 8-tandem repeats of the glutelin antisense gene, the following primers were used:
5'-AGTGGGCTGCAGGAATTCGATATCAAGCTT-3' (SEQ ID NO:3)
5'-AGTACATAGCAGCAAAACAT-3' (SEQ ID NO:4)
Similarly, for amplification of the full-length glutelin A antisense gene, the following primers were used:
5'-TACATAGCTTTAACTGATAATCTGA-3' (SEQ ID NO:5)
5'-AGTACATAGCAGCAAAACAT-3' (SEQ ID NO:6)

Northern analysis was performed according to a conventional procedure after total RNA (20 µg) was separated by electrophoresis on 1% agarose gel. The total RNA was prepared, for this analysis, from immature seeds 8 days after flowering of the transformant which was confiremed to have the 8 tandem-repeats of the glutelin A antisense gene, or those of the control plant that was transformed with the hygromycin resistance gene alone. The probe was the 5'-upstream 312 bps sequence derived from glutelin A cDNA.

The probe, (25 ng of a DNA fragment) was labeled with ³²P by using Ready Prime DNA Labeling System (Amersham), before the Northern analysis.

### (6) Production of transformants with 8 tandem-repeats of the glutelin A antisense gene

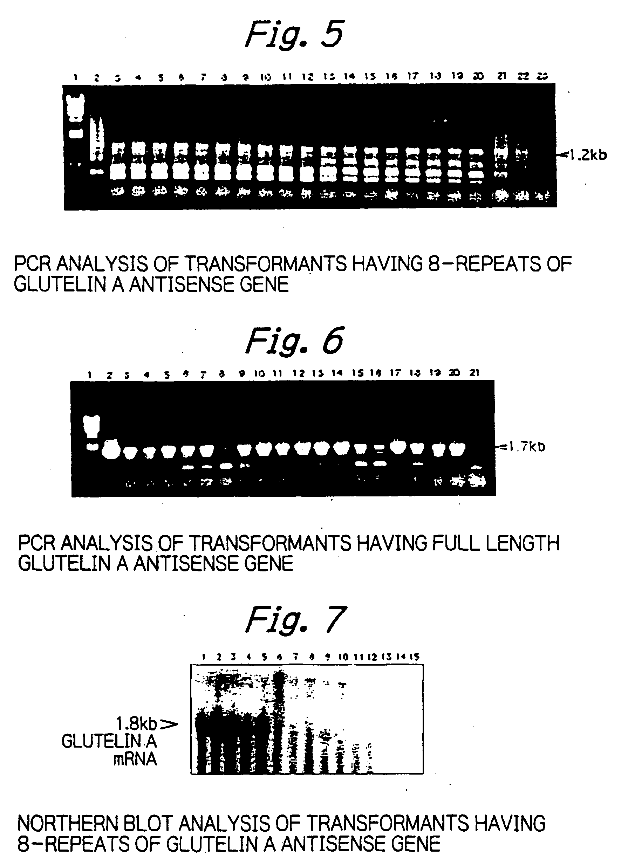

Individual 20 redifferentiated lines were obtained by regeneration of hygromycin-resistant calluses that had been transformed with the 8-tandem repeats of glutelin A antisense gene. Transformation by the antisense gene was confirmed by PCR followed by agarose electrophoresis as shown in Fig. 5. A band appeared, as expected, at the position of approximately 1.2 kbps, in the lane where the plasmid containing the antisense gene was used as a template in the PCR (Fig. 5, lane 2). All 20 transformed lines gave the same band at 1.2 kbps (Fig. 5, lanes 3-22). No such band was detected when the DNA, from untransformed lines, was used for a template in the PCR reaction (Fig. 5, lane 23).

Accordingly, we concluded that the above 20 transformants carried the 8-tandem repeats of the glutelin antisense gene.

### (7) Production of transformants with full-length glutelin antisense gene

Individual 18 redifferentiated plant lines were obtained by regeneration from hygromycin-resistant calluses that had been transformed with the full-length glutelin A antisense gene. Fig. 6 shows the results of agarose electrophoresis after PCR to confirm the introduction of the antisense gene. A band appeared, as expected at the position of approximately 1.7 kbps, in the lane where the plasmid containing the antisense gene was used as a template (Fig. 6, lane 2). All 18 transformants gave the same band at 1.7 kbps (Fig. 6, lanes 3-20). No such band was detected when the DNA, from untransformed lines, was used for a template in the PCR reaction (Fig. 6, lane 21).

Accordingly, we concluded that the above 18 transformants carried the full-length glutelin A antisense gene.

### (8) Analysis of transcription product in immature seeds from transformed plants

Fig. 7 shows the results of Northern analysis for control five lines and ten transformed lines. The transformed lines carrying the 8-tandem repeats of the glutelin A antisense gene produced apparently less amount of glutelin A mRNA (Fig. 7, lanes 6-15) than the control five lines (Fig. 7, lanes 1-5). Table 1 shows the intensity of signals as determined by Bio-imaging analyzer BAS1000 (Fuji Film Co.). The results are expressed in percentage of the mean value from the five control lines. Thus, we confirmed that the content of glutelin A mRNA was significantly lower in immature seeds of the transformants, by the effect of the introduced 8-tandem repeats of the glutelin antisense gene.

**Table 1**

| Level of transcription product in immature seeds | | | | | | | | | | |
|---|---|---|---|---|---|---|---|---|---|---|
| | Lane number | | | | | | | | | |
| Control | 6 | 7 | 8 | 9 | 10 | 11 | 12 | 13 | 14 | 15 |
| 100 | 31.1 | 11.9 | 13.1 | 11.7 | 10.0 | 7.7 | 8.2 | 3.8 | 3.0 | 1.2 |

### (9) Measurement of protein content in the seeds

Unpolished seeds from self-fertilized rice plants were ground and the proteins were extracted from 50 mg of the powder. The proteins were separated by electrophoresis on 14% polyacrylamide gel, visualized by Coomassie blue staining, and measured with a densitometer Model GS-670 (Bio-Rad Laboratories) for relative glutelin content to that of control plants.

The results were expressed in percentage of the glutelin content in self-fertilized seeds of untransformed, control rice plants (Tables 2 and 3).

The glutelin content was decreased to 63.2%, on average, by the transformants with the 8-tandem repeats of the glutelin A antisense gene and to 75.4% by those with the full-length glutelin A antisense gene.

Moreover, statistical analysis confirmed that the glutelin content was significantly lower in the transformants with the 8-tandem repeats of the glutelin A antisense gene than those with the full-length glutelin A antisense gene (p < 0.10).

**Table 2**

| Glutelin level in transformed plants (percentage of control) Transformants with 8-tandem repeats of glutelin antisense gene | | | |
|---|---|---|---|
| Line number | Glutelin content | Line number | Glutelin content |
| 1 | 59.9 | 11 | 66.0 |
| 2 | 66.9 | 12 | 76.2 |
| 3 | 42.3 | 13 | 48.1 |
| 4 | 42.6 | 14 | 83.4 |
| 5 | 66.6 | 15 | 71.7 |
| 6 | 77.0 | 16 | 98.2 |
| 7 | 39.7 | 17 | 67.8 |
| 8 | 40.1 | 18 | 61.0 |
| 9 | 55.5 | 19 | 93.2 |
| 10 | 46.1 | 20 | 61.2 |

**Table 3**

| Glutelin content in transformed plants (percentage of control) Transformants with full-length antisense gene of glutelin A | | | |
|---|---|---|---|
| Line number | Glutelin content | Line number | Glutelin content |
| 1 | 95.2 | 10 | 77.9 |
| 2 | 38.9 | 11 | 59.3 |
| 3 | 107.2 | 12 | 60.3 |
| 4 | 56.3 | 13 | 41.2 |
| 5 | 96.2 | 14 | 96.2 |
| 6 | 110.6 | 15 | 60.5 |
| 7 | 73.6 | 16 | 114.3 |
| 8 | 72.1 | 17 | 47.4 |
| 9 | 66.6 | 18 | 83.0 |

### Example 3. Production and analysis of transgenic plants with both glutelin A and glutelin B antisense genes

### (1) Construction of 8-tandem repeats of glutelin AB antisense gene for transformation

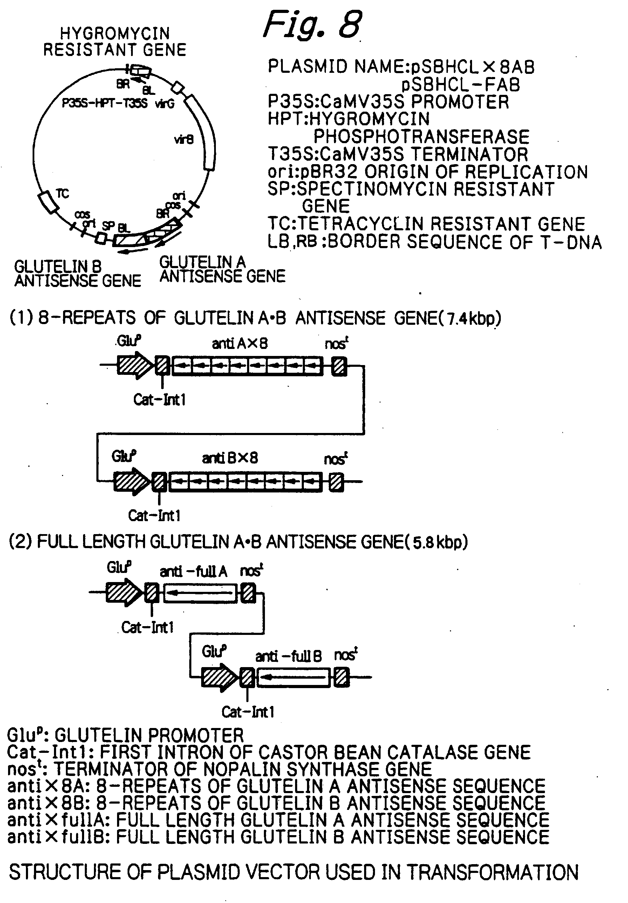

A *Sma*I/*Xba*I fragment, which contained the first intron of the castor bean catalase gene (Ohta S. et al. (1990) Plant Cell Physiol. 31, 805-813), was inserted into the *Sca*I/*Xba*I site downstream of the glutelin promoter. Then the *Xba*I/*Sph*I fragment, which contained the 8-tandem repeats of the 5'-upstream 287 bps from the full-length glutelin B cDNA, was linked downstream to the above sequence, in the antisense direction. After a *Sph*I/*Hin*dIII fragment containing the terminator of nopaline synthase was linked, the sequence was inserted into the *Hin*dIII site of plasmid vector pSBHCI x 8A, constructed in Example 2, to generate pSBHCI x 8AB (Fig. 8).

### (2) Construction of full-length glutelin AB antisense gene for transformation

A *Sma*I/*Xba*I fragment, which contained the first intron of the castor bean catalase gene (Ohta S. et al. (1990) Plant Cell Physiol. 31, 805-813), was inserted into the *Sca*I/*Xba*I site downstream of the glutelin promoter. Then, a *Sph*I/*Xba*I fragment, which contained the full-length glutelin B cDNA, was linked downstream to the above sequence, in the antisense direction. After a *Sph*I/*Hin*dIII fragment containing the terminator of nopaline synthase was linked, the sequence was inserted into the *Hin*dIII site of plasmid vector pSBHCI-FA, constructed in Example 2, to generate pSBHCI-FAB (Fig. 8).

### (3) Transformation to rice plants

A variety of the rice plant, Tsukinohikari, was transformed by *Agrobacterium tumefaciens* LBA4404 which harbored the above plasmid pSBHCI x 8AB or pSBHCIFAB, according to the method of Hiel et al. (Plant J. 6, 271-282 (1994)). Transformed calluses were selected in the presence of hygromycin as described by Hiel et al.

### (4) Measurement of protein content in seeds

Unpolished seeds from self-fertilized transformed rice plants were ground and the proteins were extracted from 50 mg of the powder. The proteins were separated by electrophoresis on 14% polyacrylamide gel, visualized by Coomassie blue staining, and the relative content of glutelin to that of control plants was measured with a densitometer Model GS-670 (Bio-Rad Laboratories).

The results were expressed in percentage of the glutelin content in the self-fertilized seeds of the untransformed, control rice plants (Table 4).

The glutelin content was reduced to 57.1%, on average, by the transformants with the 8-tandem repeats of glutelin AB antisense gene and to 69.3% by those with the full-length glutelin AB antisense gene.

Moreover, statistical analysis confirmed that the glutelin content was significantly lower in the transformants with the 8-tandem repeats of glutelin AB antisense gene than those with the full-length glutelin AB antisense gene (p<0.10).

**Table 4**

| Glutelin content | |
|---|---|
| | Mean |
| Untransformed lines | 100 |
| Transformed lines with 8-tandem repeats of glutelin AB antisense gene | 57.1 |
| Transformed lines with the full-length glutelin AB antisense gene | 69.3 |

### Example 4. Production and analysis of transformant with glutelin A antisense gene

### (1) Construction of 2-tandem repeats and 4-tandem repeats of glutelin A antisense gene for transformation

A *Sma*I/*Xba*I fragment, which contained the first intron of the castor bean catalase gene (Ohta S. et al. (1990) Plant Cell Physiol. 31, 805-813), was inserted into the *Sca*I/*Xba*I site downstream of the glutelin promoter. Then, a *Xba*I/*Sca*I fragment, which contained 2-tandem repeats or 4-tandem repeats of the 5'-upstream 312 bps from the full-length glutelin A cDNA, was linked downstream to the above sequence, in the antisense direction. After a *Sca*I/*Eco*RI fragment containing the terminator of nopaline synthase was linked, the sequence was inserted into the transformation plasmid vector with the hygromycin resistance gene, thereby to generate pSBHCI x 2A and pSBHCI x 4A, respectively.

### (2) Transformation to rice plants

A variety of the rice plant, Tukinohikari, was transformed by *Agrobacterium tumefaciens* LBA4404 which harbored the above plasmid pSBHCI x 2A or pSBHCI x 4A, according to the method of Hiel et al. (Plant J. 6, 271-282 (1994)). Transformed calluses were selected in the presence of hygromycin as described by Hiel et al. (*ibid*).

### (3) Measurement of protein content in seeds

Unpolished seeds of self-fertilized transgenic rice plants were ground and the proteins were extracted from 50 mg of the powder. The proteins were separated by electrophoresis on 14% polyacrylamide gel visualized by Coomassie blue staining, and measured with a densitometer Model GS-670 (Bio-Rad) for the relative content of glutelin to that of control plants. The result was expressed in percentage of the glutelin level in the self-fertilized seeds of the untransformed, control rice plants (Tables 5 and 6).

The glutelin content was decreased to 71.7%, on average, by the transformants with the 2-tandem repeats of the glutelin A antisense gene and to 71.5% by those with the 4-tandem repeats of the glutelin A gene.

**Table 5**

| Glutelin content in transgenic plants (percentage of control) Transformants with 2-tandem repeats of the glutelin A antisense gene | |
|---|---|
| Line number | Glutelin content |
| 1 | 66.5 |
| 2 | 66.2 |
| 3 | 67.5 |
| 4 | 93.8 |
| 5 | 64.2 |
| 6 | 60.6 |
| 7 | 75.0 |
| 8 | 79.5 |
| Mean | 71.7 |

**Table 6**

| Glutelin content in transgenic plants (percentage of control) Transformants with 4-tandem repeats of the glutelin A antisense gene | |
|---|---|
| Line number | Glutelin content |
| 1 | 54.7 |
| 2 | 89.0 |
| 3 | 93.9 |
| 4 | 64.1 |
| 5 | 68.0 |
| 6 | 66.7 |
| 7 | 67.0 |
| 8 | 73.1 |
| 9 | 67.0 |
| Mean | 71.5 |

### EFFECT OF THE INVENTION

The antisense nucleotide sequence in the present invention represents a single copy of tandem repeats of the structural gene of interest or a fragment thereof which are successively linked in the antisense direction. The antisense RNA transcribed from the antisense nucleotide sequence consists of plural repeats of the RNA sequence each being complementary to the corresponding part of the mRNA of the target protein. Therefore, the antisense RNA has a higher ability to hybridize with the mRNA and to suppress the in vivo expression of the protein. Thus, the antisense RNA of the invention containing such tandem repeats is superior to an antisense RNA without any such repeats in suppressing the synthesis of target proteins.

The description has been made mainly on the basis of suppression to the synthesis of plant proteins. Needless to say, however, the antisense sequence of the present invention is useful for suppressing the synthesis of animal proteins such as specific milk proteins or specific proteins for the purpose of treating immunological or pathogenic diseases.

## Claims

1. An antisense nucleotide sequence comprising two or more successive repeats of the tructural gene of interest or a fragment thereof in the antisense direction.

2. An antisense nucleotide sequence as claimed in Claim 1, which comprises two or more successive repeats of the sequence of a 5' region of the desired gene of interest.

3. An antisense nucleotide sequence as claimed in Claim 2, wherein the sequence of 5' region comprises at least 45 nucleotides.

4. An antisense nucleotide sequence as claimed in Claim 2, wherein the sequence of 5' region comprises at least 300 nucleotides.

5. An antisense nucleotide sequence as claimed in any of Claims 1 to 4, which comprises at least 4 repetitions of the structural gene of interest or a fragment thereof.

6. An antisense nucleotide sequence as claimed in any of Claims 1 to 4, which comprises at least 8 repetitions of the structural gene of interest or a fragment thereof.

7. An antisense nucleotide sequence as claimed in any of Claims 1 to 6, which comprises different sets of two or more successive repeats of the tructural gene of interest or a fragment thereof in the antisense direction.

8. An antisense nucleotide sequence as claimed in any of Claims 1 to 7, wherein said gene of interest is a gene coding for a storage protein of a plant seed.

9. An antisens nucleotide sequence as claimed in Claim 8, wherein the storage protein is glutelin A or gluterin B.

10. An expression vector comprising an antisense nucleotide sequence of any of Claims 1-9.

11. A transgenic host which has been transfromed by an expression vector of Claim 10.

12. A transgenic host of Claim 11, which is a plant.

13. A transgenic host of Claim 11 or 12, which is a rice plant.

14. A method for suppressing the in vivo expression of the protein of interest encoded by a structural gene comprising introducing an antisense nucleotide sequence of any of Claims 1-9 against said structural gene into the genomic gene of a target cell.
